Europäisches Patentamt

**European Patent Office**

Office européen des brevets

(19)

(11) Publication number: **0 207 888**

**A1**

(12) **EUROPEAN PATENT APPLICATION**

(21) Application number: **86810225.2**

(22) Date of filing: **23.05.86**

(51) Int. Cl.⁴: **A 61 K 9/44**
**A 61 K 31/48**

(30) Priority: **04.06.85 GB 8514089**

(43) Date of publication of application:
**07.01.87 Bulletin 87/2**

(84) Designated Contracting States:
**AT BE CH DE FR GB IT LI LU NL SE**

(71) Applicant: **SANDOZ AG**
**Lichtstrasse 35**
**CH-4002 Basel(CH)**

(84) Designated Contracting States:
**BE CH FR GB IT LI LU NL SE**

(71) Applicant: **SANDOZ-PATENT-GMBH**
**Humboldtstrasse 3**
**D-7850 Lörrach(DE)**

(84) Designated Contracting States:
**DE**

(71) Applicant: **SANDOZ-ERFINDUNGEN**
**Verwaltungsgesellschaft m.b.H.**
**Brunner Strasse 59**
**A-1235 Wien(AT)**

(84) Designated Contracting States:
**AT**

(72) Inventor: **Flury, Urs**
**23 Madlenweg**
**CH-4402 Frenkendorf(CH)**

(72) Inventor: **Lenkeit, Detlef**
**73 Riehenstrasse**
**CH-4103 Bottmingen(CH)**

(72) Inventor: **Tanner, Markus**
**125 Hörnliallee**
**CH-4125 Riehen(CH)**

(72) Inventor: **Thommen, Werner**
**2 Siegmattstrasse**
**CH-4460 Gelterkinden(CH)**

(72) Inventor: **Züger, Othmar**
**13 Heuwinkelstrasse**
**CH-4123 Allschwil(CH)**

(54) **Pharmaceutical tablet.**

(57) A tablet one or both faces of which comprise at least one pair of planes which are inclined from the tablet edge inwardly with respect to the opposite face and towards each other at an obtuse angle and which is scored with a narrow groove at the intersection of the planes characterised in that the edge of each scored face is chamferred over its entire circumference. The tablet design facilitates division thereof.

## PHARMACEUTICAL TABLET

The present invention concerns a pharmaceutical tablet provided with means to facilitate division thereof.

Compressed tablets are widely employed in the pharmaceutical industry for the administration of unit dosage forms particularly in view of their patient acceptability. The desire to optimise the dosage regimes for individual patients and to provide a high degree of flexibility when prescribing led to the development of divisible tablets.

These avoid the need to provide large numbers of individual dosage forms with their accompanying requirements for registration, production etc.

When preparing such forms the individual parameters of ease of divisibility, mechanical stability, consistency of content in divided sections play an important role.

- Ease of divisibility

Tablets must be so designed that they will divide easily on application of a relatively low degree of pressure and this is usually achieved by the use of one or more score lines on the face of the tablet. As the size of tablets is limited, further improvements were made to facilitate breakage particularly by the old or infirm e.g. by pushing on a hard flat surface. These include making the face opposed to the scored face convex or inclining the scored face towards the score lines.

- Mechanical stability

The means provided to facilitate division should be such that the overall mechanical stability of the tablets is not detrimentally affected. Thus score lines should be chosen which do not allow accidental division e.g. during transportation. Furthermore, the more "sharp edges" which are created in tablet geometry the higher the risk of partial disentegration during production and bulk transport. This in turn causes increased work in the separation of imperfect tablets which is essential for the maintenance of consistent unit dosages.

- Consistency of division

For obvious reasons it is essential that divided dosages contain as far as possible equal amounts of active substance. Thus while even distribution of active substance throughout the tablet can be achieved by appropriate formulation techniques, jagged separation along the score lines, fragmentation or chipping at the extremities and division other than at the score line can cause considerable problems.

The effect of these three factors can be exacerbated if the formulation of the active substance is such that the compressed tablets, when formed are too friable or brittle. In such cases crushing or fragmentation and chipping can occur on division.

Numerous solutions to these problems have been offered. Examples are Hager's Handbuch der Pharmazeutischen Praxis 1971, v. 7A; GB Patent 983810; GB Patent 1387643; GB Patent 1368574; CA Patent 746477; DE Patent 1200790; DE Patent 1810037.

Only a few of these solutions have proved effective and thus commercially viable and even these are restricted to active substances without formulation problems and to relatively large tablets.

With regard to formulation problems various active substances are limited in the choice of suitable excipients and those available often result in tablets which do not readily lend themselves to division. An example of such an active substance is bromocriptine where suitable excipients are silicic acid, silicium dioxide, disodium edetate dihydrate, magnesium stearate, maleic acid, corn starch, lactose(also as filler) etc.

We have now found that according to the invention bromocriptine tabletted in a form which facilitates division when pressure is applied to the tablet resting on a hard surface allows for provision of acceptable divided dosages.

With regard to patient acceptability relatively small tablets forms. are desirable which however result in the above-mentioned disadvantages when got up in a form designed for sub-division.

0207888

The tablets according to the invention provide a generally applicable solution to this problem.

We have now found that these problems can be optimally solved according to the invention by providing a tablet, one or both faces of which comprises at least one pair of planes which are inclined from the tablet edge inwardly with respect to the opposite face and towards each other at an obtuse angle and which is scored with a narrow groove at the intersection of the planes characterised in that the edge of each scored face is chamferred over its entire circumference.

The invention concerns more particularly a tablet of circular cross section, one or both faces of which comprises at least one pair of planes which are inclined from the tablet edge inwardly with respect to the opposite face and towards each other at an obtuse angle and which is scored with a narrow diametrical groove at the intersection of the planes characterised in that the edge of each scored face is chamferred over its entire circumference.

Preferably, although not essentially, all inclined surfaces including the pairs of planes, the sides of the groove(s) and the chamferred edges will incline linearly.

Angles of inclination will be chosen according to the particular active substance formulation, the number of divided sections required and the method of production.

For example the angle of inclination of the planes is ca. 170-150°, e.g. ca. 160° to each other i.e. ca. 5 to 15° to the plane of the cross section. The angle of chamferring can for example be ca. 20 to 40°, e.g. ca. 30° with respect to the plane of the cross section. The groove can be of obtuse or acute angle, e.g. 90° and its depth can be e.g. equal to that of the chamferring. The depth of the groove at its base is preferably no more than one-third the total depth of the tablet at the middle.

The tablet geometry is symmetrical about centre of the tablet. Whilst both faces can bear inclined planes, grooves and/or chamferring tablets are preferred where one face is substantially planar with chamferred edges. Tablets are preferred which have two pairs, or more preferably one pair of inclined planes. Ratio of tablet depth to diameter is e.g. 1:5 to 1:2, especially ca. 1:3.

Examples of tablet diameter are e.g. 6 to 9 mm.

Tablets are divided e.g. by resting them scored face downwards on a hard surface and applying pressure from above e.g. with finger or thumb.

Excipients will be chosen in conventional manner in accordance with the nature of the active substance and the dosage level and regime for the tablet.

Production is carried out in conventinal manner on tabletting machines fitted with appropriate punches.

Examples of active substances which can be tabletted in this way include Bromocriptine (or its mesylate), Pindolol (optionally with Clopamide or Hydrochlorothiazide), Bopindolol, Digoxin, Metolazone. Bromocriptine (or its mesylate) is particularly preferred. A particular embodiment will be described with reference to the attached drawing.

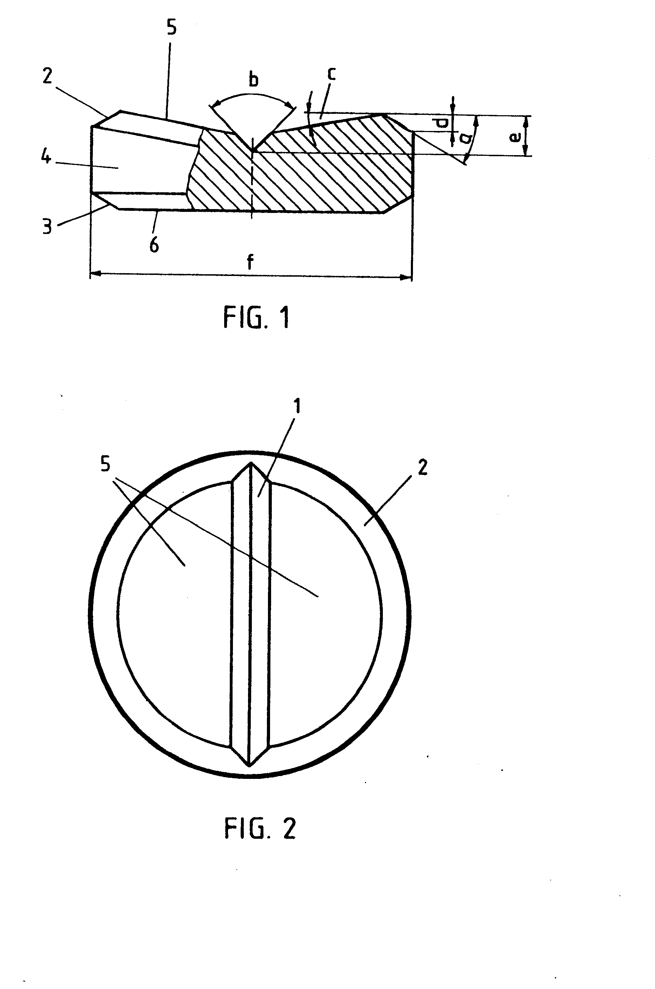

Figure 1 shows a side elevation of a tablet in partial cross section.
Figure 2 shows a plan view of a tablet.

The tablet 4 has a face 6 which is substantially planar and champferred at its edge 3. The opposite face comprises inclined planes 5 terminating at their intersection in a groove 1. The groove bearing face is also champferred 2.

Examples of angles a, b and c are 30° ± 3°; 90° ± 3° and 10° ± 3°, respectively.

Examples of distances d, e, f are shown below:

| d(mm) | e(mm) | f(mm) |
|-------|-------|-------|
| 0.28  | 0.81  | 7     |
| 0.32  | 0.92  | 8     |
| 0.36  | 1.04  | 9     |

Division is effected by placing the tablet grooved face downwards on a hard surface and applying pressure on face 6.

## Example

0207888

A typical tablet formulation employed is as follows:

| | | |
|---|---|---|
| Total | 140.000 | pts |
| Bromocriptine mesylate | 2.870 | " |
| Silicium dioxide | 0.350 | " |
| Magnesium stearate | 0.700 | " |
| Maleic acid | 1.000 | " |
| Cornstarch | 18.200 | " |
| Lactose | 116.880 | " |

This may be tabletted in conventional manner using appropriate dies in a form as described in the particular embodiment.

- Mechanical stability

| | |
|---|---|
| Transport test in bulk | : no defects/breakages |
| Packaging test | : no defects/breakages |
| "Push-out" test from blister packs | : no defects/breakages |

- Consistency of division

| | |
|---|---|
| Weight of tablet | : 140 mg |
| Weight of halves | : 70 mg |
| Discrepancy | : standard deviation 4% |

117-6671

**0207888**

WE CLAIM

1. A tablet one or both faces of which comprise at least one pair of planes which are inclined from the tablet edge inwardly with respect to the opposite face and towards each other at an obtuse angle and which is scored with a narrow groove at the intersection of the planes characterised in that the edge of each scored face is chamferred over its entire circumference.

2. A tablet of circular cross section one or both faces of which comprise at least one pair of planes which are inclined from the tablet edge inwardly with respect to the opposite face and towards each other at an obtuse angle and which is scored with a narrow diametrical groove at the intersection of the planes characterised in that the edge of each scored face is chamferred over its entire circumference.

3. A tablet according to Claim 1 or 2 one face of which comprises a pair of inclined planes.

4. A tablet according to Claim 3 wherein the angle of the planes is ca. 160° to each other; the chamferring is angled at ca. 30° to the plane of the cross section; the groove has a base angle of ca. 90° and the geometry is symmetrical about the centre.

5. A tablet according to any one of Claims 1 to 4 the depth of the groove at its base is no more than one-third of the total tablet depth.

6. A tablet according to any one of Claims 1 to 5 wherein the face opposite the scored face is substantially planar with a chamferred circumferential edge.

7. A tablet containing bromocriptine mesylate and got up in a form which facilitates division when pressure is applied thereto on a hard surface.

117-6671

0207888

8. A tablet according to Claim 6 got up in a form according to any one of Claims 1 to 6.

9. A tablet substantially as hereinbefore described with reference to the drawings and/or the examples.

FIG. 1

FIG. 2

| DOCUMENTS CONSIDERED TO BE RELEVANT | | | | EP 86810225.2 |
|---|---|---|---|---|
| Category | Citation of document with indication, where appropriate, of relevant passages | | Relevant to claim | CLASSIFICATION OF THE APPLICATION (Int. Cl.4) |
| X | AT - B - 249 875 (UCB) <br> * Claims 1-3; fig. 1,3 * | | 1-3,5, 6,8,9 | A 61 K 9/44 <br> A 61 K 31/48 |
| Y | * Claims 1-3; fig. 1,3 * | | 7 | |
| Y | DE - A - 2 351 609 (SANDOZ AG) <br> * Example 1 * | | 7 | |
| | GB - A - 993 291 (THOMAS LEE COOPER) | | | |
| X | * Claims 1,2,4,16; fig. 12,13 * | | 1-6,8,9 | |
| Y | * Claims 1,2,4,16; fig. 12,13 * | | 7 | |
| D,A | DE - A - 1 810 037 (J.R. GEIGY AG) | | | TECHNICAL FIELDS SEARCHED (Int. Cl.4) <br><br> A 61 K 9/00 <br> A 61 K 31/00 |

The present search report has been drawn up for all claims

| Place of search | Date of completion of the search | Examiner |
|---|---|---|
| VIENNA | 03-09-1986 | MAZZUCCO |